# EUROPÄISCHE PATENTANMELDUNG

(11) **EP 2 606 902 A1**
(43) Veröffentlichungstag der Anmeldung: **26.06.2013**
(21) Anmeldenummer: 11194249.6
(22) Anmeldetag: 19.12.2011
(51) Int. Cl.: A61K 36/22, A23L 1/00, A61P 1/00

(54) **Extrakt aus Rhus copallina als Arzneimittel**

(71) Anmelder: Bionorica SE, 92318 Neumarkt (DE)
(72) Erfinder: Abhau, Annette, 28857 Syke (DE)
(74) Vertreter: Simandi, Claus

(57) **Zusammenfassung**

Gegenstand der Erfindung ist ein Arzneimittel umfassend ein Extrakt hergestellt durch Extraktion der Blätter von Rhus copallina mit einem Gemisch aus Alkohol und Wasser.

## Beschreibung

Die Erfindung betrifft einen Extrakt aus den Blättern von Rhus copallina L. und ein daraus erhältliches Arzneimittel oder Nahrungsergänzungsmittel.

Es besteht ein großer Bedarf an neuen Arzneimitteln oder Nahrunsgergänzungsmitteln zur Behandlung von gastrointestinalen Erkrankungen.

Die Pflanzengattung Rhus gehört mit 150 bis 250 Arten zur Familie der Anacardiaceae. Viele Arten der Gattung Rhus sind von wirtschaftlicher Bedeutung bei der Herstellung von Gerb-und Färbemitteln. Alle Arten sind mehr oder weniger giftig. Viele Rhus Arten können Hautreizungen hervorrufen.

US 531,610 offenbart die Verwendung von wasserabweisenden Deckschichten und Decklacken, die das Harz und/oder den Saft von Rhus copallina enthalten.

US 7,528,232 B2 offenbart Verfahren und Stoffzusammensetzungen, die die Vermehrung von Pathogenen verhindern. Dabei werden einzelne Proteine, die aus Pflanzen extrahiert werden, als Antibiotikum gegen humane und tierische Pathogene eingesetzt. Gemäß US 7,528,232 können entsprechende endogene Proteine aus Pflanzen, beispielsweise aus Rhus copallina, durch Screeningverfahren erhalten werden. Ein konkretes Verfahren für Rhus copallina oder ein konkretes Protein aus Rhus copallina werden nicht offenbart.

US 7,501,557 B 1 betrifft ein System, mit dem Proteine und Proteinprodukte an die Oberfläche von Pflanzen transportiert werden und ein Verfahren, um Proteine aus der Pflanze abzusondern. US 7,501,557 B 1 schlägt vor, dazu beispielsweise Rhus copallina mit dem in US 7,501,557 B 1 offenbarten Genkonstrukt zu versehen.

Die Dissertation von Annette Abhau zum Thema "The survival of a Malagasy lemur species Propithecus verreauxi coquereli in captivity : The vital role of a self-selected plant diet" (freigeschaltet am 20.06.2007; http://deposit.d-nb.de/cgi-bin/dokserv?idn=984680519 beschäftigt sich u.a. mit der genauen qualitativen und quantitativen Analyse der Pflanze Rhus copallina als essentielles Nahrungsmittel für die genannte Lemurenart, auch als Sifakas bezeichnet. In der Doktorarbeit wurden für die Analysen und Untersuchungen die stiellosen Blätter verwendet. Der Nährstoffgehalt der Blätter wurde nach einer Methode von Weender bestimmt. Zur Analyse der Inhaltsstoffe von Rhus copallina wurden die Blätter von Rhus copallina mit einem Gemisch aus 80 % Methanol und 20 % Wasser (V/V) extrahiert. Vor der präparativen Isolierung einzelner Verbindungen mit Hilfe einer Polyamidsäule und nachfolgender präparativer HPLC-Technik wurden die zerkleinerten Blätter mit n-Hexan präextrahiert.

Für eine gravimetrische Isolierung wurde Isobutylmethylketon und Wasser verwendet.

John K. Crellin and Jane Philpott (A Reference Guide to Medicinal Plants, 1989 Duke University Press, 2. Auflage 1997, Seiten 417 - 419) geben einen Überblick über die Verwendung von verschiedenen Rhus-Arten als Hausmittel.

Für R. glabra L. und R. copallina sind beschrieben
a) die Verwendung als Arzneitee, der durch Kochen der Beeren in Wasser hergestellt wird, als Hustensaft, zum Gurgeln und für die Nieren;
b) die Verwendung der Blätter äußerlich bei Schwellungen und Geschwüren sowie als Duft-und Geschmacksstoff für Tabak und als Farbe, und
c) die Verwendung der Beeren (im Mixer zerkleinert, angefärbt und mit Wasser und Zucker versetzt) als Limonadenersatz und bei Erkältung.

John K. Crellin and Jane Philpott beschreiben ebenfalls die medizinische Verwendung von Rhus coriaria und der verwandten Cotinus coggygria Scop. als Bindemittel, zur Trocknung und bei Erkältung. Diese Anwendungen sind bereits seit dem Jahr 1597 bekannt. Für Rhus coriaria ist auch die Verwendung für "fluxes of the belly" und Diarrhö bekannt. Ferner wird die Verwendung als Fiebermittel erwähnt, sowie die kühlenden und adstringierenden Eigenschaften der Beeren. Auch die adstringierenden Eigenschaften der Blätter, Wurzeln und Beeren von R. coriaria, deren Verwendung bei rauhem Hals und Affektionen der Mundschleimhaut, bei Diarrhö, Verbrennungen und Hämorrhoiden im Zusammenhang mit R. coriaria, sowie die Verwendung der Blätter und Beeren von R. glabra bei gastrointestinalen Beschwerden gehören zur traditionellen Anwendung dieser Rhus-Arten.

Rhus glabra, Rhus typhina und Rhus aromatica sind als traditionelle nordamerikanische Medizin bekannt, und zwar als Adstringens, bei Verdauungsbeschwerden, zum Gurgeln und Mundspülen, sowie bei Beschwerden von Haut und Blase. Als Pflanzenteile werden hier nicht die Blätter verwendet, sondern nur die Rinde, Wurzeln und Früchte (In "Medicinal Plants of the world" von Ben-Erik van Wyk und Michael Wink).

Von einigen Arten (beispielsweise Toxicodendron-Arten) werden Hautreizungen hervorgerufen.

Es ist die Aufgabe der vorliegenden Erfindung ein neues Arzneimittel bereitzustellen. Gegenstand der Erfindung ist ein Arzneimittel oder Nahrungsergänzungsmittel enthaltend wässrig-alkoholische Extrakte aus Rhus copallina L. und deren Verwendung als Arzneimittel oder Nahrungsergänzungsmittel.

Gegenstand der Erfindung ist insbesondere ein Arzneimittel umfassend einen Extrakt hergestellt durch Extraktion der Blätter von Rhus copallina L. mit einem Gemisch aus Alkohol und Wasser.

Gegenstand der Erfindung ist insbesondere ein Nahrungsergänzungsmittel umfassend einen Extrakt hergestellt durch Extraktion der Blätter von Rhus copallina L. mit einem Gemisch aus Alkohol und Wasser.

Gegenstand der Erfindung ist ebenfalls eine pharmazeutische Zusammensetzung umfassend einen Extrakt hergestellt durch Extraktion der Blätter von Rhus copallina L. mit einem Gemisch aus Alkohol und Wasser und gegebenenfalls weitere Hilfs- und Zusatzstoffe.

Ein Alkohol-Wasser Extrakt aus Blättern von Rhus copallina wurde als Arzneimittel oder Nahrungsergänzungsmittel bisher nicht verwendet oder beschrieben.

In bevorzugten Ausführungsformen der Erfindung wird der Extrakt dadurch hergestellt, dass die Blätter von Rhus copallina L. mit Ethanol und Wasser extrahiert werden. In einer anderen bevorzugten Ausführungsform der Erfindung wird der Extrakt dadurch hergestellt, dass die Blätter von Rhus copallina L. mit Methanol und Wasser extrahiert werden. Die Extraktion kann beispielsweise einem Gemisch, das mindestens 20 % oder 30 % Ethanol/Methanol umfasst, vorzugsweise 40 % oder 50 % Ethanol/Methanol, besonders bevorzugt 60 %, 70 % oder 80% Ethanol oder Methanol. Eine besonders bevorzugte Ausführungsform der Erfindung betrifft ein Arzneimittel oder Nahrungsergänzungsmittel, wobei die Extraktion mit einem Gemisch aus Alkohol und Wasser durchgeführt wird, beispielsweise einem Gemisch mit mindestens 85 % Ethanol oder Methanol, vorzugsweise 90 % oder 95 % Ethanol oder Methanol oder mehr, bis hin zur Verwendung von reinem Ethanol oder Methanol für die Extraktion. In einer besonderen Ausführungsform der Erfindung kann das Extraktionsmittel neben Ethanol und Wasser noch bis zu 10 % andere Lösungsmittel enthalten, beispielsweise Ethylacetat.

Das Arzneimittel oder Nahrungsergänzungsmittel ist geeignet für Tiere, insbesondere Nutztiere, und vorzugsweise Menschen. Eine bevorzugte Ausführungsform der Erfindung betrifft das Arzneimittel oder Nahrungsergänzungsmittel zur (spezifischen) Anwendung am Menschen. Eine weitere besondere Ausführungsform betrifft das Arzneimittel zur (spezifischen) Anwendung am Menschen, die unter chronischen Magen- und/oder Darmfunktionsstörungen leiden.

Die Erfindung betrifft daher die Anwendung des Arzneimittels zur Behandlung von gastrointestinalen Erkrankungen, insbesondere chronischen Magen- und/oder Darmfunktionsstörungen umfassend die Regulation des Magen-Darm Traktes, bei allgemeinen Verdauungsproblemen, beispielsweise gegen Blähungen, gegen Koliken und/oder gegen Verstopfung, bei Magenproblemen, wie Völlegefühl und Unwohlsein, Magen- und/oder Bauchschmerzen.

Eine weitere besondere Ausführungsform der Erfindung betrifft die spezifische Anwendung bei Kindern ab dem Säuglingsalter. Eine weitere besondere Ausführungsform der Erfindung betrifft die Anwendung bei Menschen, die immundefizient sind, an einer bakteriellen Fehlbesiedlung des Darmes leiden oder deren Erkrankung mit einer erhöhten gastrointestinalen Permeabilität assoziiert ist beispielsweise entzündliche Darmerkrankungen wie M. Crohn, Colitis ulcerosa, Diabetes, Asthma, allergische Hauterkrankungen, atopische Dermatitis (Neurodermitis).

Besonders bevorzugte Ausführungsformen der Erfindung betreffen Arzneimittel zur (spezifischen) Anwendung als Antidiarrhoikum, Antiphlogistikum, Analgetikum, Antibiotikum, Antimykotikum, Mittel zur Regeneration der Schleimhaut, Mittel zur Juckreizstillung, Adstringens, Hämostyptikum, zur Behandlung von atopischer Dermatitis (Neurodermitis), Spasmolytikum.

Überraschenderweise wurde gefunden, dass ein Extrakt, insbesondere ein wäßrigalkoholischer Extrakt aus den Blättern von Rhus copallina antiphlogistische, antiinflammatorische (entzündungshemmende), juckreizstillende, adstringierende, antiseptische und antibakterielle Eigenschaften aufweist.

Ein weiterer Gegenstand der Erfindung ist insbesondere die (spezifische) Anwendung des Arzneimittels oder Nahrungsergänzungsmittels zur Behandlung von gastrointestinalen Erkrankungen.

Die Erfindung betrifft die Anwendung des Arzneimittels oder Nahrungsergänzungsmittels zur Behandlung und Prophylaxe von gastrointestinalen Erkrankungen, umfassend chronisch-entzündliche Darmerkrankungen wie Morbus Crohn, Colitis ulcerosa, Diarrhö, Gastroenteritis, Hämorrhoidalleiden, Reizdarm, eine bakterielle Fehlbesiedlung mit resistenten und/oder fakultativ und/oder obligat pathogenen Keimen. Die Erfindung betrifft auch die Anwendung des Arzneimittels oder Nahrungsergänzungsmittel zur Toxinbindung, insbesondere bei Exotoxinen wie beispielsweise dem aus Vibrio cholerae stammenden Choleratoxin und auch gegenüber anderen toxinproduzierenden enteropathogenen Bakterien, beispielsweise EHEC.

Die Erfindung betrifft die Anwendung des Arzneimittels oder Nahrungsergänzungsmittel zur Reduktion einer erhöhten gastrointestinalen Permeabilität.

Die erfindungsgemäßen (spezifischen) Anwendungen umfassen die Verwendung des Arzneimittels zur Behandlung und/oder Prophylaxe und/oder Nachsorge, der jeweils genannten Indikationen.

Die Erfindung betrifft ebenfalls die (spezifische) Anwendung des Arzneimittels oder Nahrungsergänzungsmittels bei Krebs und Krebsvorstufen im Gastrointestinaltrakt, wie Darmkrebs oder Magenkrebs und bei Keimen mit krebsauslösendem Potential wie Helicobacter pylori.

Daher betrifft ein weiterer Gegenstand der Erfindung die Eradikationstherapie mittels dem erfindungsgemäßen Arzneimittel.

Die Erfindung betrifft die (spezifische) Anwendung gegenüber bestimmten Problemkeimen wie dem methicillinresistenten Staphylococcus aureus und anderen Staph. aureus Stämmen, sowie Pseudomonas aeruginosa, die insbesondere bei immungeschwächten und hospitalisierten Patienten zum vitalen Problem werden können.

Die Erfindung betrifft die (spezifische) Anwendung gegenüber auf der Haut und/oder Schleimhaut vorkommenden Keimen, beispielsweise dem Corynebacterium acnes.

Die Definitionen und Begriffe "Magen- und Darmfunktionsstörungen, chronisch-entzündliche Darmerkrankungen, Diarrhö, Gastroenteritis, Hämorrhoidalleiden, Reizdarm, Morbus Crohn, Cholera, Verdauungsprobleme, wie Blähungen, Koliken und/oder gegen Verstopfung, Magenproblemen, wie Völlegefühl und Unwohlsein, Magen- und/oder Bauchschmerzen" können beispielsweise dem Klinischen Wörterbuch Pschyrembel, Auflage 2012, De Gruyter Verlag, Berlin entnommen werden.

Das Arzneimittel kann oral, rektal, dermal oder mucosal dargereicht werden. Eine besonders bevorzugte Ausführungsform der Erfindung betrifft die orale, dermale oder mukosale Darreichung bzw. Applikation des Arzneimittels.

Gegenstand der Erfindung ist auch ein Verfahren zur Herstellung eines Extrakts aus Rhus copallina, wobei die Blätter von Rhus copallina geerntet, gereinigt und zerkleinert werden und die Blätter dann mit einem Gemisch aus Wasser und Alkohol, beispielsweise Ethanol oder Wasser extrahiert werden, beispielsweise mit einem Gemisch mit mindestens 40 % Alkohol, vorzugsweise 50 % Alkohol oder mehr, besonders bevorzugt 60 % oder 80 % Alkohol der mehr. In einer besonderen Ausführungsform der Erfindung kann das Extraktionsmittel neben Ethanol und Wasser noch bis zu 10 % andere Lösungsmittel enthalten.

Die Extraktion mit Alkohol und Wasser ist besonders vorteilhaft, weil dadurch aus der Vielzahl der Inhaltsstoffe in den Blättern von Rhus copallina nur die Inhaltsstoffe extrahiert und angereichert werden, die besonders vorteilhafte pharmakologische Eigenschaften haben. Eine Übersicht über die Vielzahl der chemischen Verbindungen in Rhus copallina sowie den Gehalt an diesen Stoffen in der Pflanze kann der oben genannten Dissertation von Annette Abhau entnommen werden. Durch das erfindungsgemäße Extraktionsverfahren werden aus diesen vielen Inhaltsstoffen, die in den Blättern vorhanden sind, nur bestimmte Inhaltsstoffe in bestimmten Mengen extrahiert. Diese Extraktion führt daher zu einer Zusammensetzung des Extrakts, welches die vorteilhaften pharmakologischen Eigenschaften aufweist, insbesondere die antiphlogistischen, anti-inflammatorischen, juckreizstillenden, adstringierenden, antiseptischen und antibakteriellen Eigenschaften sowie die vorteilhaften gastrointestinalen Wirkungen. Zu letzterem zählt die darmwandabdichtende Wirkung, die Erniedrigung einer erhöhten gastrointestinalen Permeabilität, eine Verlangsamung der Darmpassage, eine Erhöhung der Stuhlkonsistenz, die Verminderung einer gastrointestinalen Hypersekretion und toxinbindende Eigenschaften gegenüber enteropathogenen Keimen.

Die Blätter von Rhus copallina werden im Frühling oder im Sommer oder im Herbst geerntet. Gegebenenfalls kann auch eine Mischung von Blättern, die zu verschiedenen Jahreszeiten geerntet werden, verwendet werden. Beispielsweise können Blätter verwendet werden, die im Mai, Juni und/oder Oktober geerntet werden. Die Blätter können frisch verwendet werden, oder vor der Extraktion durch Trocknung haltbar gemacht werden. Es werden vorzugsweise nur die stiellosen Blätter ohne Stengel bzw. Zweige verwendet.

Die getrockneten oder frischen Blätter werden zerkleinert (geschnitten) und gegebenenfalls pulverisiert und gesiebt. Die zerkleinerten Blätter werden mit dem Extraktionsmittel versetzt. Die Extraktion wird über einen bestimmten Zeitraum und unter bestimmten Bedingungen durchgeführt. Durch die Extraktion werden die gewünschten Inhaltsstoffe aus den Blättern von Rhus copallina herausgelöst und angereichert, während unerwünschte Begleitstoffe in den Blättern verbleiben. Anschließend wird der Rückstand aus Blättern abgetrennt und es verbleibt der Rhus copallina Extrakt.

Gegenstand der Erfindung ist ebenfalls ein Extrakt aus Rhus copallina, der nach diesem Verfahren hergestellt wird.

Die Extraktion kann beispielsweise mittels Mazeration oder Perkolation erfolgen. Bei der Mazeration werden die Blätter mit dem Extraktionsmittel eine definierte Zeit stehen gelassen, bis ein Gleichgewichtszustand erreicht ist (keine erschöpfende Extraktion). Bei der Perkolation werden die Blätter kontinuierlich mit dem Extraktionsmittel versetzt und der entstandene Extrakt abgetrennt.

Eine Ausführungsform der Erfindung betrifft Rhus copallina Fluidextrakt, wobei die Extraktion für maximal 7 Tage, vorzugsweise 1 bis 5 oder 6 Tage, besonders bevorzugt 2, 3 oder 4 Tage durchgeführt wird oder gegebenenfalls andere bekannte Mazerationsverfahren.

Besonders bevorzugt ist eine kurze und schnelle Extraktion, da auf diese Weise die in den Blättern enthaltenen Gerbstoffe nicht zu Gallussäure abgebaut werden. Eine schnelle Extraktion kann beispielsweise innerhalb von 10 Minuten oder bis zu 5 Stunden, vorzugsweise innerhalb einer Zeit von 30 Minuten und 3 Stunden, besonders bevorzugt etwa 1 Stunde durchgeführt werden. Besonders geeignete Reaktionsbedingungen für kurze und schnelle Extraktionsverfahren sind dem Fachmann bekannt.

Eine bevorzugte Ausführungsform der Erfindung betrifft die Extraktion der Blätter bei erhöhter Temperatur, beispielsweise bei 30°C, 35°C, 40 °C oder 45 °C. Dadurch kann die Extraktionszeit verkürzt werden. Beispielsweise wird die Extraktion bei erhöhter Temperatur und unter Rückfluss für 2 oder 3 Stunden durchgeführt.

Nach der Extraktion wird der Rückstand (die Blätter) abgetrennt und der Extrakt gegebenenfalls eingeengt bzw. aufkonzentriert. Die Aufkonzentrierung kann beispielsweise durch schonendes Rotationsverdampfen bei 30 °C bis 40 °C erfolgen. Durch das Rotationsverdampfen kann der Extrakt bis fast zur Trockne eingeengt werden. Das Volumen des Extrakts kann beispielsweise um ein Drittel oder die Hälfte oder noch weiter reduziert werden.

Um den mengenmäßigen Bezug zwischen eingesetzten Blättern (Droge) und Extrakt deutlich zu machen, wird für einen Extrakt das Droge-Extrakt-Verhältnis angegeben. Das Droge-Extrakt-Verhältnis (DEV) gibt an, welche Ausgangsmenge an Droge (Drogengewicht, Trockenmasse) für die Bereitung einer bestimmten Menge des Extrakts eingesetzt wurde. Mit dem DEV kann bei der Dosierung immer auf die eingesetzte Drogenmenge umgerechnet werden und verschiedene Extrakte der Droge können in ihrer Qualität verglichen werden und Rückschlüsse auf die Anreicherung der Inhaltsstoffe gezogen werden.

Aus dem Extrakt von Rhuis copallina können beispielsweise Trockenextrakte z.B. für Tabletten hergestellt werden oder Fluidextrakte. Fluidextrakte (Flüssigextrakte) von Rhus copallina haben beispielsweise ein Verhältnis von Extraktivstoffen aus einem Teil Droge zu der Menge an Flüssigextrakt von 1:1. Fluidextrakte sind flüssige Drogenzubereitungen, bei denen für die Extraktion der Droge möglichst wenig Extraktionsflüssigkeit eingesetzt wird.

Bei dem verwendeten Extrakt aus den Blättern von Rhus copallina wird beispielsweise ein Droge / Extraktionsmittel-Verhältnis von 20 mg Droge zu 1 ml Lösungsmittel bzw. Extraktionsmittel, oder 15 g Droge in 150 ml Extraktionsmittel verwendet. In den Blättern waren je nach Jahreszeit allein bis zu 28 % Gerbstoffe (m/m) enthalten. Dadurch kann das DEV entsprechend variiert werden. Das DEV kann beispielsweise bei 3 : 1 bis 5 : 1 liegen, andere DEV sind erfindungsgemäß ebenfalls umfasst.

Ein weiterer Gegenstand der Erfindung ist ebenfalls eine pharmazeutische Zusammensetzung, vorzugsweise in Form einer Salbe, einem Saft, einer Lotion, einer Lösung, einem Suppositorium, welche einen wässrig/alkoholischen Extrakt aus den Blättern von Rhus copallina umfasst. Die Salbe, Lotion, Lösung, Saft oder das Suppositorium können neben dem wässrig/alkoholischen Extrakt die üblichen Träger, Hilfs- und Zusatzstoffe umfassen.

Die erfindungsgemäßen pharmazeutischen Zusammensetzungen oder Zubereitungen können in Form von Dosierungseinheiten hergestellt werden. Dies bedeutet, dass die Zusammensetzungen in Form einzelner Teile, vorzugsweise Kapseln, Dragees und Ampullen vorliegen, deren Wirkstoffgehalt an dem erfindungsgemäßen Extrakt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge des erfindungsgemäßen Extrakts (Wirkstoff), die bei einer Applikation verabreicht wird und die gewöhnlich einer ganzen, einer halben, einem Drittel oder einem Viertel einer Tagesdosis entspricht.

In einer weiteren bevorzugten Ausführungsform kann die galenische Formulierung einer Calziummanteltablette gewählt werden, wie in EP1392337 offenbart.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Das erfindungsgemäße Nahrungsergänzungsmittel kann ebenfalls in Form einer Zusammensetzung verabreicht werden, wie oben beschrieben. In einer weiteren Ausführungsform kann das Nahrungsergänzungsmittel einem beliebigen Nahrungsmittel oder Lebensmittel, einschließlich Wasser, zugesetzt werden.

Nahrungs- und Lebensmittel im Sinne dieser Erfindung sind insbesondere solche, wie nicht abschließend in EU-Richtlinie 2002/46/EG vom 10. Juni 2002 definiert.

## Patentansprüche

1. Arzneimittel umfassend einen Extrakt hergestellt durch Extraktion der Blätter von Rhus copallina L. mit einem Gemisch aus Alkohol und Wasser.

2. Arzneimittel nach Anspruch 1, wobei der Alkohol Ethanol oder Methanol ist.

3. Arzneimittel nach einem der vorhergehenden Ansprüche zur Anwendung am Menschen.

4. Arzneimittel nach einem der vorhergehenden Ansprüche zur Anwendung als Antidiarrhoikum, Antiphlogistikum, Antiseptikum, Antibiotikum, insbesondere bei bakterieller gastrointestinaler Fehlbesiedlung und/oder zur Bekämpfung von Problemkeimen im Haut- und Schleimhautbereich, zur Regeneration von Haut und Schleimhaut, zur Juckreizstillung, Adstringens, Hämostyptikum, bei atopischer Dermatitis (Neurodermitis), Spasmolytikum.

5. Arzneimittel nach einem der vorgehenden Ansprüche zur Anwendung bei gastrointestinalen Erkrankungen, insbesondere zur Behandlung und/oder Prophylaxe von gastrointestinalen Erkrankungen.

6. Arzneimittel nach einem der vorgehenden Ansprüche, wobei gastrointestinale Erkrankung ausgewählt ist aus der Gruppe
a.) chronischen Magen- und/oder Darmfunktionsstörungen, wie die Regulation des Magen-Darm Traktes, allgemeine Verdauungsprobleme, Blähungen, Koliken und/oder gegen Verstopfung, Magenprobleme, wie Völlegefühl und Unwohlsein, Magen-und/oder Bauchschmerzen,
b.) chronisch-entzündliche Darmerkrankungen wie Morbus Crohn oder Colitis ulcerosa, Diarrhö, Gastroenteritis, Hämorrhoidalleiden, Reizdarm, Bindung bzw. Neutralisation von Toxinen enteropathogener Keime, insbesondere Choleratoxin und verwandte Toxine, zur Reduktion einer erhöhten gastrointestinalen Permeablilität und damit assoziierten Erkrankungen,
c.) Krebs und Krebsvorstufen im Gastrointestinaltrakt, wie Darmkrebs oder Magenkrebs
d.) zur Eradikationstherapie von Helicobacter pylori.

7. Arzneimittel nach einem der vorgehenden Ansprüche zur oralen, dermalen und/oder mucosalen Darreichung.

8. Salbe, Saft, Lotion oder Zäpfchen, insbesondere gerbstoffhaltig, umfassend einen Extrakt aus den Blättern von Rhus copallina L. und gängigen Hilfs- und Zusatzstoffen.

9. Verfahren zur Herstellung eines Extrakts aus Rhus copallina, wobei die Blätter von Rhus copallina L. geerntet, gereinigt und zerkleinert werden und die Blätter dann mit einem Gemisch aus Wasser und Alkohol, vorzugsweise Ethanol extrahiert werden, beispielsweise einem Gemisch mit mindestens 40 % Alkohol, vorzugsweise 50 % Alkohol oder mehr, besonders bevorzugt 60 % bis 80 % Alkohol oder mehr.

10. Extrakt aus Rhus copallina L. hergestellt oder erhältlich durch ein Verfahren nach Anspruch 9.

11. Pharmazeutische Zusammensetzung umfassend einen Extrakt hergestellt durch Extraktion der Blätter von Rhus copallina L. mit einem Gemisch aus Alkohol und Wasser und gegebenenfalls weitere Hilfs- und Zusatzstoffe.

12. Nahrungsergänzungsmittel umfassend einen Extrakt hergestellt durch Extraktion der Blätter von Rhus copallina L. mit einem Gemisch aus Alkohol und Wasser.
